# EUROPEAN PATENT SPECIFICATION

(11) **EP 2 521 459 B1**
(45) Date of publication and mention of the grant of the patent: **07.02.2018**
(21) Application number: 10728913.4
(22) Date of filing: 09.06.2010
(51) Int. Cl.: A23K 10/18, A23K 50/80

(54) **METABOLITES IN ANIMAL FEED**
METABOLITEN IN TIERFUTTER
MÉTABOLITES DANS UNE ALIMENTATION ANIMALE

(30) Priority: 10.08.2009 MY 0907013
(43) Date of publication of application: 14.11.2012
(73) Proprietor: Universiti Putra Malaysia (UPM), 43400 Selangor, Darul Ehsan (MY)
(72) Inventor: FOO, Hooi Ling, 43400 Selangor Darul Ehsan (MY); LOH, Teck Chwen, 43400 Selangor Darul Ehsan (MY); KARUNAKARAMOORTHY, Anuradha, 43400 Selangor Darul Ehsan (MY); SHAMSUDIN, Mariana Nor, 43400 Selangor Darul Ehsan (MY); HAJI ABDUL RAHIM, Raha, 43400 Selangor Darul Ehsan (MY)
(74) Representative: Krauss, Jan
(86) International application number: PCT/MY2010/000095
(87) International publication number: WO 2011/019264

(56) References cited:
- US-A1- 2002 176 910
- SON V M ET AL: "Dietary administration of the probiotic, Lactobacillus plantarum, enhanced the growth, innate immune responses, and disease resistance of the grouper Epinephelus coioides" FISH AND SHELLFISH IMMUNOLOGY, ACADEMIC PRESS, LONDON, GB LNKD- DOI:10.1016/J.FSI.2009.02.018, vol. 26, no. 5, 3 March 2009 (2009-03-03), pages 691-698, XP026097888 ISSN: 1050-4648 [retrieved on 2009-03-03]
- LOH T C ET AL: "Effects on growth performance, faecal microflora and plasma cholesterol after supplementation of spray-dried metabolite to postweaning rats" CZECH JOURNAL OF ANIMAL SCIENCE, POTRAVINARSKYCH INFORMACI, PRAGUE, vol. 54, no. 1, 1 January 2009 (2009-01-01), pages 10-16, XP009122277 ISSN: 1212-1819
- FOO, H.L.; LOH, T.C.; LAI, P.W.; LIM, Y.Z.; KUFLI, C.N.; RUSUL, G.: "Effects of Adding Lactobacillus plantarum I-UL4 Metabolites in Drinking Water of Rats" PAKISTAN JOURNAL OF NUTRITION, vol. 2, no. 5, 1 September 2003 (2003-09-01), pages 283-288, XP002601023
- VENDRELL D ET AL: "Protection of rainbow trout (Oncorhynchus mykiss) from lactococcosis by probiotic bacteria" COMPARATIVE IMMUNOLOGY, MICROBIOLOGY AND INFECTIOUS DISEASES, PERGAMON PRESS, OXFORD, GB LNKD- DOI:10.1016/J.CIMID.2007.04.002, vol. 31, no. 4, 1 July 2008 (2008-07-01), pages 337-345, XP022688792 ISSN: 0147-9571 [retrieved on 2008-05-27]
- FOO H L ET AL: "Effects of feeding Lactobacillus plantarum I-UL4 isolated from Malaysian tempeh on growth performance, faecal flora and lactic acid bacteria and plasma cholesterol concentrations in postweaning", FOOD SCIENCE AND BIOTECHNOLOGY, THE KOREA SOC. OF FOOD SCIENCE AND TECHNOLOGY, vol. 12, no. 4, 1 January 2003 (2003-01-01), pages 403-408, XP009122258, ISSN: 1226-7708
- BALCAZAR J L ET AL: "Characterization of probiotic properties of lactic acid bacteria isolated from intestinal microbiota of fish", AQUACULTURE, ELSEVIER, AMSTERDAM, NL, vol. 278, no. 1-4, 10 June 2008 (2008-06-10), pages 188-191, XP022669418, ISSN: 0044-8486, DOI: 10.1016/J.AQUACULTURE.2008.03.014 [retrieved on 2008-03-18]

## Description

### FIELD OF INVENTION

The present invention relates to a fish feed product comprising probiotic bacterial strain, wherein bacterial strain is *Lactobacillus plantarum* I-UL4 strain and deposited under the accession number 36838 at BIOTEC Culture Collection, for use in reducing faecal *A*. *hydrophila* count in fish. Preferred embodiments of the invention are described in dependent claims 2 to 7..

### BACKGROUND OF INVENTION

LAB are used extensively in the food industry in the manufacturing of fermented products including milk products such as e.g. yoghurt and cheese, meat products, bakery products, wine and vegetable products. *Lactococcus lactis* is one of the most commonly used LAB in dairy starter cultures. LAB are commonly used as inoculants in feedstuffs of plant and animal origin, i.e. for preservation purposes.

The LAB used consist one or several of the LAB species that normally used in the handling of foodstuffs, for example, streptococci type N, pediococci, leconostoc or lactobacilli. They can be added in the form of pure cultures but it is preferable to use mixed cultures from a fermented material of vegetable or animal origin. Probiotic LAB are also known to produce a wide variety of antibacterial substances, as well as inhibitory primary metabolites, such as acetic acid, lactic acid, propionic acid, ethanol, diacetyl, hydrogen peroxide, bacteriocins and antiobiotic-like substances with activity against Gram negative and Gram positive bacteria. Bacteriocins are proteinaceous compounds, which have antimicrobial properties that able to inhibit many different bacterial species, especially pathogenic bacteria. This compound has received vast attention because they are produced by beneficial to human health bacteria and also often used as natural food bio-preservatives. It has been shown that administration of bacteriocins influences the bacterial ecology of the gastrointestinal tract and reduces the levels of pathogenic bacteria in different parts of gastrointestinal tract. Although there are tremendous reports for the health benefits of LAB, only limited evidence that LAB metabolites could provide beneficial effects to the host are available. The metabolites of environmental friendly LAB exhibiting antagonistic effects against pathogenic bacteria may be of applied interest as feed supplement by replacing the use of antibiotics. There is an ever-increasing demand for aquaculture products and a similar increase in the search for alternatives to antibiotics. Aquaculture has become one of the fastest-growing food-producing sectors, and there has been much focus on the possibility of applying conventional methods known from livestock farming for disease control and growth promotion in aquaculture. Disease outbreaks caused by the presence of harmful organisms in the surrounding environment is recognized as one of the most significant constraints on aquaculture production, affecting the economic development of the sector in many countries.

There has been much focus on the control of the presence of pathogens in the aquaculture environment by applying and testing conventional methods used in the farming of terrestrial animals. The methods include the addition of antimicrobial compounds, such as e.g. antibiotics, to the environment. However, these conventional approaches have had limited success. As expected, the need for disease control has been accounted for by the development of novel disease control methods. One such method comprises the use of "probiotics", which are antagonistic bacterial strains to be used for the control of populations of potential pathogens through e.g. competitive exclusion. This technology has been used in the farming of terrestrial animals, where commercial probiotic products have been launched and available in the market. Oral administration of these products induces increased resistance to enteric infections and enhances the general immune response of the treated animal.

Moreover, antibiotic and probiotics are the common feed supplements used in aquaculture activity. Probiotic is defined as any live microbial adjunct that has a beneficial effect on the host by changes in the host-related or ambient microbial community, through an improvement in the use of feed or its nutritional value, or by enhancing the host response to disease or by improving the quality of its environment. Growth promoting antibiotic is another common feed additives, mainly due to their positive effects on growth or feed conversion efficiency and the reduction of incidence of certain diseases. However, the extensive use of antibiotic may lead to the development of resistance in a number of pathogenic bacteria species. Likewise, cross-resistance may occurs to therapeutic antibiotic belongs to the same class of drug, particularly those with close relationships with human antimicrobial therapies. Some countries already imposed restrictions or prohibitions on the use of antibiotics as growth promotants and this have drawn attention to possible alternatives.

The application of LAB metabolites in aquaculture could increase the production of aquatic animals to support the ever increasing demands of consumers and give an impact on reducing the usage of antibiotic as feed additives that would result the resistance against pathogenic bacteria. In comparison to live microorganism, metabolites have advantages in storing, transporting and handling.

The prior art within aquaculture presently shows that bacterial species have been tested for control of the aquatic environment. WO02/00035 describes a bioactive food complex for controlling bacterial diseases in aquatic animals. The food complex is described as an emulsion comprising at least one probiotic bacteria, selected for antimicrobial chemical production, and at least one inhibitory or regulatory compound. The at least one inhibitory or regulatory compound is a furanone, which comprises a group of chemical compounds that inhibit surface colonisation of microorganisms in general. A generically described probiotic bacteria is Bacillus. No specific preferred Bacillus strains are mentioned and the working examples just refer to probiotic bacteria as such.

JP2000103740A describes a medicine for fish and shellfishes, which contains metabolites and products as an active principle, such as a peptide obtained by the mixed culture of *Bacillus thuringiensis* and *Bacillus pumilu.* Based on specific examples it is concluded that this mixed culture improves immune system activation and prevents infection for cultured fishes. The problem to be solved by the present invention is addressed by an aquatic animal (e.g. fish) feed product capable of significantly increasing the resistance of aquacultured animal (e.g. fish) against relevant pathogenic microorganisms. The solution is based on that the present inventors identification of a fish feed product enriched with metabolites derived from probiotic LAB as feed supplement for aquaculture animals that is capable of significantly increasing the resistance of aquacultured fish against relevant pathogenic microorganisms.

SON V M ET AL, "Dietary administration of the probiotic, Lactobacillus plantarum, enhanced the growth, innate immune responses, and disease resistance of the grouper Epinephelus coioides", FISH AND SHELLFISH IMMUNOLOGY, ACADEMIC PRESS, LONDON, GB LNKD- DOI:10.1016/J.FSI.2009.02.018, vol. 26, no. 5, recommend a dietary *L. plantarum* administration to promote growth and enhance immunity in the grouper *Epinephelus coioides.*

LOH T C ET AL, "Effects on growth performance, faecal microflora and plasma cholesterol after supplementation of spray-dried metabolite to postweaning rats", CZECH JOURNAL OF ANIMAL SCIENCE, POTRAVINARSKYCH INFORMACI, PRAGUE, (20090101), vol. 54, no. 1, describe the effects of spray dried LAB metabolites on various parameters in post-weaning rats.

US 2002/0176910 A1 discloses animal feed products comprising sorbic acid and live or dead micro-organisms producing bacteriocins.

FOO, H.L.; LOH, T.C.; LAI, P.W.; LIM, Y.Z.; KUFLI, C.N.; RUSUL, G., "Effects of Adding Lactobacillus plantarum I-UL4 Metabolites in Drinking Water of Rats", PAKISTAN JOURNAL OF NUTRITION, (20030901), vol. 2, no. 5, pages 283 - 288, describe the effect of LAB metabolites in drinking water on various parameters in rats.

VENDRELL D ET AL, "Protection of rainbow trout (Oncorhynchus mykiss) from lactococcosis by probiotic bacteria", COMPARATIVE IMMUNOLOGY, MICROBIOLOGY AND INFECTIOUS DISEASES, PERGAMON PRESS, OXFORD, GB LNKD- DOI:10.1016/J.CIMID.2007.04.002, vol. 31, no. 4, show that probiotic supplementation reduced fish mortality significantly in rainbow trout.

FOO HL et al. "Effects of feeding Lactobacillus plantarum I-UL4 isolated from Malaysian Tempeh on growth performance, faecal flora and lactic acid bacteria and plasma cholesterol concentrations in postweaning rats" Food science and biotechnology 12(4):403-408 · August 2003, study the effects of oral administration of *L. plantarum* I-UL4 (LP) in the diet of postweaning rats on growth performance, faecal pH, Enterobacteriaceae and lactic acid bacteria (LAB) counts and plasma cholesterol concentrations.

José L. Balcázar, Daniel Vendrell, Ignacio de Blas, Imanol Ruiz-Zarzuela, José L. Muzquiz, Olivia Girones, "Characterization of probiotic properties of lactic acid bacteria isolated from intestinal microbiota of fish", Aquaculture, Volume 278, Issues 1-4, 10 June 2008, Pages 188-191, analyse the effects of probiotic bacteria against the adhesion of fish pathogens.

### SUMMARY OF THE INVENTION

The present invention relates to fish feed product comprising probiotic bacterial strain, wherein bacterial strain is *Lactobacillus plantarum* I-UL4 strain and deposited under the accession number 36838 at BIOTEC Culture Collection, for use in reducing faecal *A*. *hydrophila* count in fish..
It is said that the animal feed comprising metabolites produced from the *Lactobacillus plantarum* I-UL4 strain and the metabolites includes bacteriocins, vitamin B and organic acids, such as formic acid, acetic acid and lactic acid.

Preferably, the animal feed comprises metabolites in a concentration of at least 0.1% to 0.5% of dry weight. Indeed the metabolites are used into the feed as an additive or supplement.

Moreover, said fish feed is capable of providing between 20% and 50% increase on animal growth and capable of providing at least 80% increase in relative survival rate. Furthermore, the fish feed is capable of reducing faecal *Enterobacteriaceae* and *A*. *hydrophila* count and increasing faecal LAB count in aquatic animals.

Also disclosed is a method of performing aquaculture of aquatic animals wherein the aquatic animals are in contact with an aquatic animal feed product comprising a probiotic *Lactobacillus plantarum* bacteria. The aquatic animals are in contact with an amount of aquatic animal feed that corresponds from 5% to about 20% of a total feed intake of the aquatic animals of the aquaculture.

Further disclosed is a method for feeding an aquatic animal present in an aquaculture comprising feeding the aquatic animal with an aquatic animal feed product comprising a probiotic *Lactobacillus plantarum* bacterium.

In addition, the present disclosure shows an aquatic animal feedstuff produced by the above preceding methods. Preferably also the disclosure describes animal feed formulation that includes total protein (between 30% and 40% of the total feed ration), total fat (total fat is between 3% and 5% of the total feed ration), moisture (between 10% and 12% of the total feed ration), and metabolite powder (preferably includes bacteriocins, combine with vitamin B, and organic acids). The formulation further includes any of the combination selected from the group of protein, fat and moisture. Indeed, the metabolite powder is between 0.1% and 0.5% of the total feed ration.

It is described that the animal feed having the means to increase a total feed intake between 3% and 10% and the formulation providing the capability to reduce faecal Enterobacteriaceae count and increase faecal lactic acid bacteria count in aquatic animals.

The present invention consists of several novel features and a combination of parts hereinafter fully described and illustrated in the accompanying description and drawings, it is understood that various changes in the details may be made without departing from the scope of the invention or sacrificing any of the advantages of the present invention.

### BRIEF DESCRIPTION OF THE DRAWINGS

The present invention will be fully understood from the detailed description given herein below and the accompanying drawings which are given by way of illustration only, and thus are not limitative of the present invention, wherein:
Figure 1 shows the agar well diffusion assay of LAB metabolite against *Aeromonas hydrophila*
Figure 2 shows the effect of LAB metabolite on survival rate of tilapia after being challenged with *Aeromonas hydrophila.* Notes: T1, LAB metabolite; T2, Control. ± indicates standard error. Values within the same row and experiment sharing a common superscript letter are not significantly different, P > 0.05.
Figure 3 shows the bacteria viable count in fish digesta fed with and without LAB metabolite. Notes: T1, LAB metabolite; T2, Control. ± indicates standard error.Values within the same row and experiment sharing a common superscript letter are not significantly different, P > 0.05.

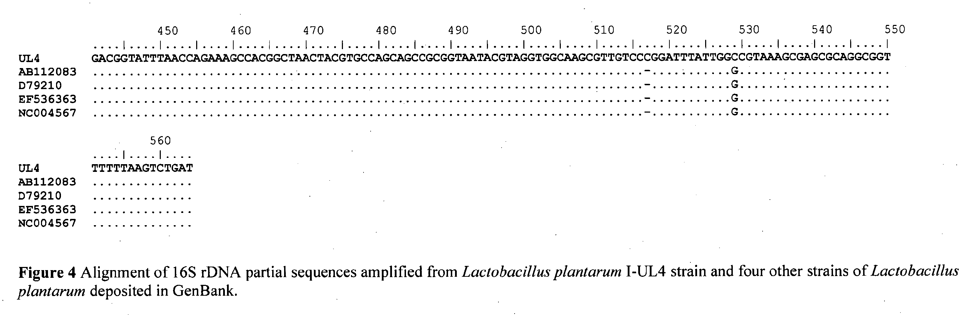

Figure 4 shows alignment of 16S rDNA partial sequences amplified from *Lactobacillus plantarum* I-UL4 strain and four other strains of *Lactobacillus plantarum* deposited in GenBank.

### DETAILED DESCRIPTION OF THE INVENTION

The present invention relates to the use of metabolites derived from probiotic LAB as feed supplement for aquaculture animals. More particularly, the present invention relates to fish supplement with improve quality, resistance to undesirable bacterial strains and to a process for preparing metabolites mixed into fish feed.

The solution is based on that the present inventors' identification of a fish feed product enriched with a metabolite derived from probiotic LAB that is capable of significantly enhanced the resistance of aquacultured fish against relevant pathogenic microorganisms. It was demonstrated that the utilization of the metabolites derived from LAB strains isolated from Malaysian foods, *Lactobacillus* sp., for improving the growth performance, immunity and overall health of aquatic animal; for the control of gastrointestinal tract pathogens including antibiotic resistance gastrointestinal tract pathogens and their associated diseases by both a reduction in the rate of colonization and the severity of the deleterious physiological effects of the colonization of the antibiotic-resistance pathogen.

Accordingly, the invention relates to a fish feed product according to claim 1. Without being limited to theory, it is believed that the metabolites derived from probiotic LAB are capable of significantly increasing the amount of aquatic animal antibodies against pathogenic microorganisms. The enhancement of antibody production renders the aquatic animal more resistant to these pathogenic microorganisms. See working examples herein for further details. It is believed to be the first time that it is demonstrated that a metabolite derived from probiotic LAB gives its positive effect through growth performance, survival rate and negative effect of pathogen in aquaculture animals such as fish.

A further advantage of a feed product comprising metabolite derived from probiotic LAB is that the growth weight gain and growth length of the aquatic animal is improved. See working examples herein for further details.

### DEFINITION

Prior to a discussion of the detailed embodiments of the invention, a definition of specific terms related to the main aspects of the invention is provided.

The term feed or feed composition means any compound, preparation, mixture, or composition suitable for, or intended for intake by an animal.

The term "aquaculture" should be understood according to the art as an aquatic culture of aquatic animals wherein the animals are cultured in a physically defined space such as, e.g., in cages or tanks.

The term "probiotic" is a well-defined term in the art and relates to a microorganism that when it has been in physical contact (e.g., when eaten) with an aquatic animal it confers health benefit to the animal.

### BEST MODE TO CARRY OUT THE INVENTION

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims. When a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. When the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited. It must be noted that as used herein and in the appended claims, the singular forms "a," "an," and "the" include plural referents unless the context clearly dictates otherwise.

### EXAMPLES

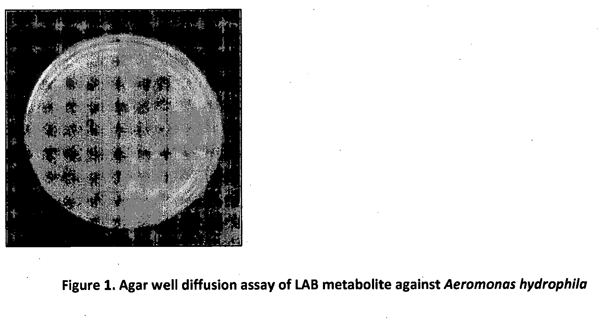

**i)** *Lactobacillus* sp., isolated from fermented tapioca (*tapai ubi)* was used in this study. These bacteria were obtained from our own collection (Department of Bioprocess Technology, Universiti Putra Malaysia). The LAB strain was kept in Man Rogosa Sharpe (MRS) broth at -20°C. The LAB strain was revived twice in MRS broth and incubated anaerobically at 20°C - 40°C before preparing the LAB metabolite. The overnight culture was inoculated into MRS broth and incubated anaerobically for overnight at 20°C - 40°C. The metabolite was collected by separating the bacterial cells with centrifugation at 8000 x g for 10 min. The metabolite was then kept at 4 °C till use. The metabolite was also spray dried.
**ii)** In the Agar-Well Diffusion Method (Tagg *et al.,* 1976), 10 - 100µl of the LAB metabolite was pipetted into the pre-punched agar well. The supernatant was allowed to diffuse for an hour. The plate was then overlaid with soft agar inoculated with the indicator organism and incubated at 30°C - 40°C overnight. Inhibition was evident by a clear zone formation around the well (**Figure 1**) of UL4 metabolite against indicator organism with minimum mean diameter of 1 cm.
**iii)** The feeding trial was conducted for 4 weeks. Two experimental groups, with LAB metabolite (T1) and without LAB metabolite (T2), each with three replicates that followed a completely randomized design were carried out by using uniform size tanks (25 L capacity). A total of 48 tilapias, with body weight of 17-18 g, were allowed to acclimatize for 2 weeks before the trial.
**iv)** The spray-dried LAB metabolite was diluted according to the pre-determined amount that gave the highest bacteriocin activity. Under sterile conditions, the diluted powder was then incorporated into commercial diet. The required amount was gently sprayed on the diet and slowly mixed. Fish fed with only commercial diet was served as control. The fish were fed ad-libitum twice daily. All fishes were weighed every week to assess the growth performance in terms of weight gain, specific growth rate (SGR) and feed conversion ratio (FCR). The feed intake was recorded daily (**Table 1**). The body weight gain was significantly higher (P<0.05) in T1 compared to control. Similar trend was also found for specific growth rate, where the growth rate for T1 group was statistically significant. T1 also had the lowest FCR, indicating the fish fed with LAB metabolite was able to convert feed into fish tissue efficiently.

**Table 1: Growth performance of fishes fed with LAB metabolite**

| **Parameters** | **T1** | **T2** |
|---|---|---|
| **Weight gain (g)** | 7.7^{a} ± 0.01 | 5.5^{b} ± 0.01 |
| **Specific growth rate (% per day)** | 1.35^{a} ± 0.01 | 1.01^{b} ± 0.01 |
| **FCR (feed conversion ratio)** | 1.63^{a} ± 0.05 | 1.89^{b} ± 0.01 |

| | | |
|---|---|---|
| Notes: T1, LAB metabolite; T2, Control. ± indicates standard erro).Values within the same row and experiment sharing a common superscript letter are not significantly different, P > 0.05. | | |

For immunological study, blood was withdrawn from the caudal vein of anaesthetized tilapia every week. The blood was stored overnight at 4 °C and the serum was collected by centrifugation at 1000 x g for 20 min before being stored at - 80 °C. The antibody levels were determined from the immunized fishes by indirect ELISA (Takemura, 1993). The 96-well ELISA plates were coated overnight at 4 °C with LAB metabolite at concentration of pre-determined amount that gave the highest activity in 100 µl well⁻¹ of coating buffer (15 mM Na₂CO₃, 35 mM NaHCO₃, pH 9.6). The plates were then washed three times with phosphate buffered saline containing 0.05 % (v/v) Tween-20 (PBS-T). The well was then blocked with 250 µl well⁻¹ of 1 % (w/v) BSA in PBS for 2 h at 22 °C. After washing three times with PBS-T, the diluted serum of tilapia at 1:4 ratio in PBS (100 µl well⁻¹) were added and incubated for 3 h at 22 °C. After washing three times with PBS-T, the plates were added with 100 µl well⁻¹ horseradish conjugated mouse anti-tilapia IgM (1:54) (Aquatic Diagnostics Ltd) and incubated for 1 h at 22 °C. After washing, the reaction was developed by the addition of tetramethylbenzidine dihydrochloride (TMB)/H₂O₂ substrate and further incubated for 10 min at 22°C. The reaction was terminated by adding 50 µl well⁻¹ of 2M H₂SO₄. Optical density was measured at 450 nm by ELISA reader (Anthos Zenyth 340 Microplate Reader) (**Table 2**). During 4 weeks of oral feeding with LAB metabolite, there was an increase of antibody levels in the fish serum. The control group showed weak or no responses. Increase in antibody levels was detected at week 2 after the fish were fed with LAB metabolite orally, and the antibody level reached its maximal level at week 3. However, the antibody level was decreased slightly after week 3, but was much higher compared to control

**Table 2: Antibody production in tilapia fed with LAB metabolite.**

| | **OD₄₅₀ₙₘ** | | |
|---|---|---|---|
| Week | T1 | | T2 |
| | 0.25^{a} ± 0.00 | | |
| 0 | 0.37^{a} ± 0.01 | | 0.27^{a} ± 0.01 |
| 1 | 0.80^{a} ± 0.06 | | 0.32^{a} ± 0.03 |
| 2 | 1.14^{a} ± 0.01 | | 0.52^{b} ± 0.05 |

| | | | |
|---|---|---|---|
| Notes: T1, LAB metabolite; T2; Control. ± indicates standard error. Values within the same row and experiment sharing a common superscript letter are not significantly different, P > 0.05. | | | |

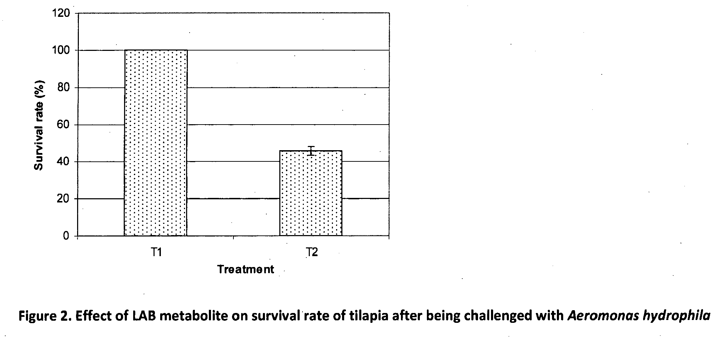

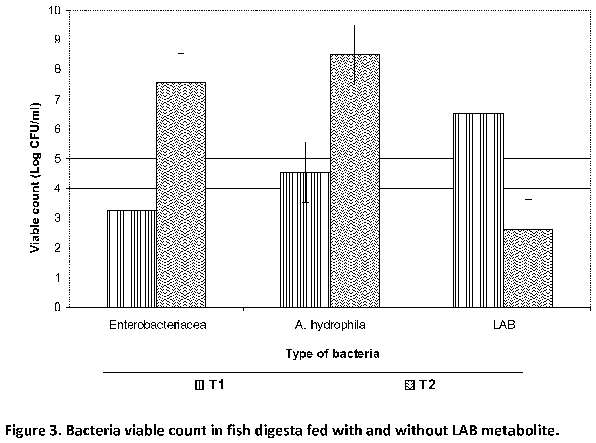

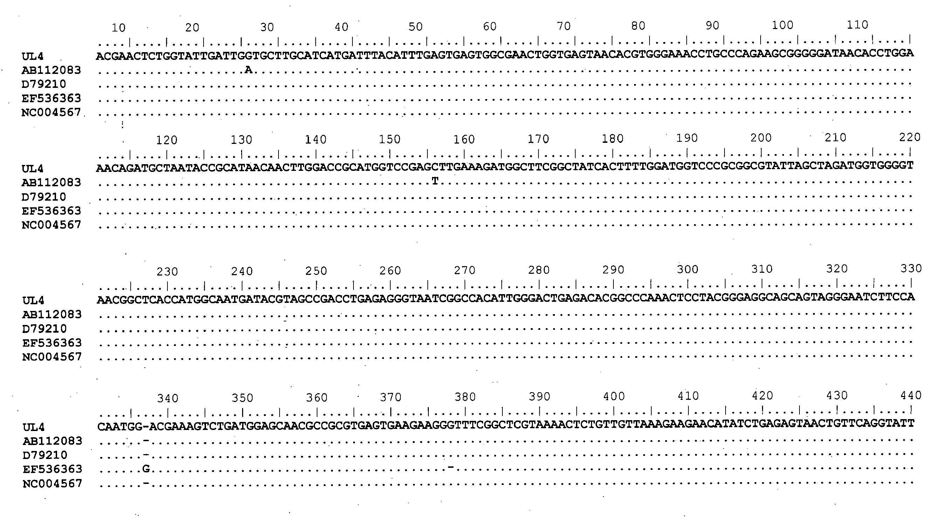

vi) After 4 weeks of feeding trial, each group was injected with 0.1 ml of 10⁸ CFU/ml of pathogenic strain of *Aeromonas hydrophila.* Mortalities were recorded and removed daily over 10 days. The cause of death was determined by culturing the kidney of morbid fish for *A*. *hydrophila.* During the experimental period, the fish was fed as usual (**Figure 2**). 100% protection and no mortality were recorded for fish immunized with LAB metabolite compared to control groups. The survival rate was statistically significant (P<0.05) in T1 in relation to T2.
vii) Analysis of the intestine digesta was done on samples consisting of excised intestines from the surviving fish from each treatment groups. The samples were then homogenized with 1 ml of sterile saline solution using a stomacher. 10-fold serial dilution (10⁻¹ to 10⁻⁸) was then carried out in triplicate and 100 µl of an appropriate dilution was plated on Eosin Methylene Blue Agar (EMB), Glutamate Starch Phenol Red Agar (GSP) and de Man, Rogosa and Sharp agar (MRS) for Enterobacteriaceae, *A*. *hydrophila* and LAB count respectively. The plates were incubated overnight at 30°C until the colonies were visible (**Figure 3**). The viable count for Enterobacteriaceae and *A*. *hydrophila* was lower in T1 compared to T2. Meanwhile, the LAB count was the highest in T1 and the lowest count in T2.
**viii)** A fish food was produced in a way corresponding to the above Example and a mixture was obtained following composition in percentages by weight of the mixture **(Table 3):**

**Table 3: Percentage composition of fish diet**

| Ingredients | % |
|---|---|
| Crude protein | 30 - 40 |
| Crude fat | 3 - 5 |
| Moisture | 10-12 |
| Metabolite powder | 0.1 - 0.5 |

## Claims

1. A fish feed product comprising probiotic bacterial strain, wherein bacterial strain is *Lactobacillus plantarum* I-UL4 strain and deposited under the accession number 36838 at BIOTEC Culture Collection, for use in reducing faecal *A*. *hydrophila* count in fish.

2. The fish feed product as claimed in claim 1, wherein the fish feed comprises metabolites produced from the *Lactobacillus plantarum* I-UL4 strain.

3. The fish feed product as claimed in claim 2, wherein the metabolites include bacteriocins, vitamin B and organic acids, such as formic acid, acetic acid and lactic acid.

4. The fish feed product as claimed in claim 1, wherein the fish feed comprises metabolites in a concentration of 0.1% to 0.5% of dry weight.

5. The fish feed product as claimed in claim 1, wherein the metabolites is used into the feed as an additive or supplement.

6. The fish feed product as claimed in claim 1, wherein the fish feed is capable of providing between 20% and 50% increase on animal growth, and/or is capable of providing at least 80% increase in relative survival rate.

7. The fish feed product as claimed in claim 1, wherein the fish feed is capable of reducing faecal enterobacteriaceae and *A*. *hydrophila* count and increasing faecal LAB count in fish.

## Patentansprüche

1. Ein Fischfutterprodukt umfassend eines probiotischen bakteriellen Stammes, wobei der bakterielle Stamm der *Lactobacillus plantarum* I-UL4 Stamm ist und unter der Hinterlegungsnummer 36838 bei der BIOTEC Culture Collection hinterlegt ist, zur Verwendung beim Verringern der fäkalen *A*. *hydrophila* Anzahl im Fisch.

2. Das Fischfutterprodukt nach Anspruch 1, wobei das Fischfutter Metabolite umfasst, die vom *Lactobacillus plantarum* I-UL4 Stamm produziert werden.

3. Das Fischfutterprodukt nach Anspruch 2, wobei die Metabolite Bakteriocine, Vitamin B und organische Säuren, wie beispielsweise Ameisensäure, Essigsäure, und Milchsäure, beinhalten.

4. Das Fischfutterprodukt nach Anspruch 1, wobei das Fischfutter Metabolite in einer Konzentration von 0,1% bis 0,5% des Trockengewichtes umfasst.

5. Das Fischfutterprodukt nach Anspruch 1, wobei die Metabolite in das Futter als ein Zusatzmittel oder Ergänzungsmittel gemischt werden.

6. Das Fischfutterprodukt nach Anspruch 1, wobei das Fischfutter fähig ist, zwischen 20% und 50% Steigerung des Tierwachstums zu erreichen, und/oder fähig ist, mindestens 80% Steigerung der relativen Überlebensrate zu erreichen.

7. Das Fischfutterprodukt nach Anspruch 1, wobei das Fischfutter fähig ist, im Fisch die fäkale Enterobacteriaceae und *A*. *hydrophila* Anzahl zu verringern und die fäkale LAB Anzahl zu erhöhen.

## Revendications

1. Produit alimentaire pour poissons comprenant une souche bactérienne probiotique, dans lequel la souche bactérienne est *Lactobacillus plantarum* I-UL4 et a été déposée sous le numéro d'accès 36838 à la BIOTEC Culture Collection, destiné à être utilisé dans la réduction du compte fécal d'*A*. *hydrophila* chez les poissons.

2. Produit alimentaire pour poissons selon la revendication 1, dans lequel l'aliment pour poissons comprend des métabolites produits à partir de la souche de *Lactobacillus plantarum* I-UL4.

3. Produit alimentaire pour poissons selon la revendication 2, dans lequel les métabolites comprennent des bactériocines, de la vitamine B et des acides organiques, tels que l'acide formique, l'acide acétique et l'acide lactique.

4. Produit alimentaire pour poissons selon la revendication 1, dans lequel l'aliment pour poissons comprend des métabolites à une concentration de 0,1 % à 0,5 % du poids sec.

5. Produit alimentaire pour poissons selon la revendication 1, dans lequel les métabolites sont utilisés dans l'aliment à titre d'additif ou de supplément.

6. Produit alimentaire pour poissons selon la revendication 1, dans lequel l'aliment pour poissons est capable de réaliser entre 20 % et 50 % d'augmentation de la croissance des animaux, et/ou est capable de réaliser une augmentation d'au moins 80 % du taux de survie relatif.

7. Produit alimentaire pour poissons selon la revendication 1, dans lequel l'aliment pour poissons est capable de réduire le compte fécal d'entérobactéries et d'*A*. *hydrophila* et d'augmenter le compte fécal de LAB chez les poissons.


### SEQUENCE LISTING

<110> Universiti Putra Malaysia
<120> METABOLITES IN ANIMAL FEED
<130> SK/PCT0017/UPM/09
<160> 5
<170> PatentIn version 3.4
<210> 1
   <211> 562
   <212> DNA
   <213> Lactobacillus plantarum
<220>
   <221> misc_feature
   <223> Lactobacillus plantarum strain I-UL4
<400> 1
   acgaactctg gtattgattg gtgcttgcat catgatttac atttgagtga gtggcgaact      60
   ggtgagtaac acgtgggaaa cctgcccaga agcgggggat aacacctgga aacagatgct     120
   aataccgcat aacaacttgg accgcatggt ccgagcttga aagatggctt cggctatcac     180
   ttttggatgg tcccgcggcg tattagctag atggtggggt aacggctcac catggcaatg     240
   atacgtagcc gacctgagag ggtaatcggc cacattggga ctgagacacg gcccaaactc     300
   ctacgggagg cagcagtagg gaatcttcca caatgggacg aaagtctgat ggagcaacgc     360
   cgcgtgagtg aagaaggttt cggctcgtaa aactctgttg ttaaagaaga acatatctga     420
   gagtaactgt tcaggtattg acggtattta accagaaagc cacggctaac tacgtgccag     480
   cagccgcggt aatacgtagg tggcaagcgt tgtccggatt tattgggcgt aaagcgagcg     540
   caggcggttt tttaagtctg at                                              562

### SEQUENCE LISTING

<110> Universiti Putra Malaysia
<120> METABOLITES IN ANIMAL FEED
<130> SK/PCT0017/UPM/09
<160> 5
<170> PatentIn version 3.4
<210> 1
   <211> 562
   <212> DNA
   <213> Lactobacillus plantarum
<220>
   <221> misc_feature
   <223> Lactobacillus plantarum strain I-UL4
<400> 1
   acgaactctg gtattgattg gtgcttgcat catgatttac atttgagtga gtggcgaact    60
   ggtgagtaac acgtgggaaa cctgcccaga agcgggggat aacacctgga aacagatgct   120
   aataccgcat aacaacttgg accgcatggt ccgagcttga aagatggctt cggctatcac   180
   ttttggatgg tcccgcggcg tattagctag atggtggggt aacggctcac catggcaatg   240
   atacgtagcc gacctgagag ggtaatcggc cacattggga ctgagacacg gcccaaactc   300
   ctacgggagg cagcagtagg gaatcttcca caatgggacg aaagtctgat ggagcaacgc   360
   cgcgtgagtg aagaaggttt cggctcgtaa aactctgttg ttaaagaaga acatatctga   420
   gagtaactgt tcaggtattg acggtattta accagaaagc cacggctaac tacgtgccag   480
   cagccgcggt aatacgtagg tggcaagcgt tgtccggatt tattgggcgt aaagcgagcg   540
   caggcggttt tttaagtctg at                                            562